# EUROPEAN PATENT APPLICATION

(11) **EP 2 075 006 A1**
(43) Date of publication of application: **01.07.2009**
(21) Application number: 08153965.2
(22) Date of filing: 02.04.2008
(51) Int. Cl.: A61K 39/12, C12N 7/02

(54) **Aquatic nervous necrosis virus (NNV) vaccine and manufacture method thereof**

(30) Priority: 20.12.2007 CN 200710302226
(71) Applicant: Schweitzer Chemical Corporation Ltd., City of Industry, CA 91748 (US)
(72) Inventor: Chi, Shau-Chi, c/o National Taiwan University, Taipei City (TW)
(74) Representative: Viering, Jentschura & Partner

(57) **Abstract**

The invention provides an aquatic nervous necrosis virus (NNV) vaccine and a method for preparing the vaccine. The method comprises Following steps: (a) culturing nervous necrosis viruses (NNV) by using a cell line to obtain a supernatant containing said nervous necrosis viruses (NNV), and (b) treating said supernatant with an inactivating agent to form an aquatic nervous necrosis virus (NNV) vaccine. Particularly suited inactivating agents are formalin and binary ethyleneimine (BEI). The aquatic nervous necrosis virus (NNV) vaccine can be further encapsulated by a nanoencapsulating process to form a nanoencapsulated aquatic nervous necrosis virus (NNV) vaccine.

## Description

### BACKGROUND OF THE INVENTION

### 1. FIELD OF THE INVENTION

The invention relates to a vaccine and methods for preparation and use thereof, and in particular, to a vaccine for aquatic animal and methods for preparation and use thereof.

### 2. DESCRIPTION OF THE PRIOR ART

Viral nervous necrosis disease (VNN disease) has been the viral disease prevailing in sea water aquaculture fishes in recent years. This disease came first in Japan, and then prevailed over the world. Excepting Africa, disease cases emerged over other continents, and became a worldwide fish viral disease. The virus causing this disease was known as Nervous necrosis virus (NNV) and referred also as Fish nodavirus or Beta-nodavirus.

Nervous necrosis virus is a single-stranded RNA virus, non-enveloped, and with icosahedron profile as well as a viral particle size of a diameter in the range of about 25 to 34 nanometers (nm). Larvae at the early stage of infection exhibited abrupt darting, darkened skin color, lost balance and abnormal swimming behaviors such as spiral swimming. Some fishes bend their bodies to one side, darken their body color, and float on water surface with one bodyside up. Severly infected larvae may drift on water surface, then sink onto the bottom and finally die off. High mortality might occur within one week after the clinical symptom appeared (Yoshikoshi, Inoue, 1990; Glazebrook *et al*., 1990; Bloch *et al*., 1991; Renault *et al*., 1991).

As the aquaculture industry advanced, traditional culture has been transforming gradually into intensive culture. Most of aquaculture countries in the world adopt intensive culture. Although intensive aquaculture is able to increase greatly the production per unit area, it may cause a number of problems, in particular, the disease eruption due to improper management during the course of aquaculture.

Controlling methods currently employed is principally drug treatment, such as, disinfection of apparatus, larvae, and sterification of fish feed with chlorine, iodine, ammonia or ozone. For preventing fish viral disease, it is important to develop fish vaccine to increase the immunity of fish. Furthermore, viral nervous necrosis disease (VNN) has occurred in Taiwan every year and caused heavy economic loss of aquatic industry. Accordingly, the development of an effective vaccine against viral nervous necrosis disease becomes the most important topic in VNN control.

### SUMMARY OF THE INVENTION

The object of the invention is to provide an aquatic nervous necrosis virus (NNV) vaccine giving a protection effect higher than a relative percentage survival (RPS) of 30%.

In order to achieve the object, the invention provides a method for preparing an aquatic nervous necrosis virus (NNV) vaccine described above, said method comprising following steps: (a) culturing nervous necrosis viruses (NNV) by using a cell line to obtain a supernatant containing said nervous necrosis viruses (NNV), and (b) treating said supernatant with an inactivating agent to form an aquatic nervous necrosis virus (NNV) vaccine.

The aquatic nervous necrosis virus (NNV) vaccine can also be prepared by the following method, said method comprising following steps: (a) culturing nervous necrosis viruses (NNV) by using a cell line to obtain a supernatant containing said nervous necrosis viruses (NNV), (b) treating said supernatant with an inactivating agent, and (c) forming an aquatic nervous necrosis virus (NNV) vaccine from said inactivated supernatant by means of a nanoencapsulating process.

Said cell line is used to amplify viruses so as to obtain a NNV-containing culture supernatant. The cell line includes all fish cell lines with good susceptibility to NNV, such as GF-1 (US pat. No. 6,436,702, ATCC deposit no. PTA-85), GF, GL, GB3 (Biochem. Biophys. Res. Comun. 2006 Oct 19), SISE (J. Virol. Methods. 2006 Nov; 137(2):309-16), GS (J. Virol. Methods. 2006 Jan; 131(1):58-64), SAF1 (Fish Shellfish Immunol. 2004 Jan; 16(1):11-24), SSN-1 (Dis. Aquat. Organ. 1999 Dec 22; 39(1):37-47), GL-av or E-11 (Dis. Aquat. Organ. 2000 Nov 14;43(2):81-9) and the like. GF-1 is the preferred cell line.

Said nervous necrosis viruses (NNV) used in the invention include all NNV isolates isolated from NNV-infected fish. These include, for example, RGNNV, SJNNV, TPNNV, BFNNV, FEV, DGNNV, GGNNV, and G9410YA, G9508KS or HGOOGD and the like. NNV isolate can be obtained from a variety of strains such as SJNNV (D30814), TPNNV (D38637), BFNNV (D38635), RGNNV (D38636), MGNNV (AF245003), DGNNV (AF245004), B00GD (AY140794), G9508KS (AY140798), HG00GD (AY140799), YP99PD (AY140800), G9410YA (AY140801), AHNNV (AF160473), AHNorA (AJ245641), ACNNV (AF445800), and the like. All of nucleotide sequences from strains mentioned above have been emcoded in Gene Bank.

Said inactivating agent is formalin or binary ethyleneimine (BEI). In the formalin inactivation process, the virus is treated with 0.1-0.2% formalin solution at 24-30°C for 3-7 days, followed by dialyzing in phosphate buffered saline (PBS) for 24 hours to form a working inactivated vaccine. In the BEI inactivation process, 2-bromoethyleneamine is added in NaOH solution, and the mixture is heated at 30-37°C for 1-2 hours to form BEI inactivating agent. Thereafter, the viruses can be treated in 3-4 mM BEI at 23-27°C for 3-5 days, followed by dialyzing in PBS for 24 hours to form a working inactivated vaccine.

In the nanoencapsulation process used in the invention, 30-99.9% by weight of a base and 0.1-70% by weight, preferably 0.1% by weight, of the inactivated vaccine are used. The base mentioned above can be a non-water soluble base consisting of hydrogenated vegetable oil, hydrogenated animal oil, or C8-64 saturated fatty acid and derivatives thereof. The substance to be encapsulated is the inactivated virus.

The inactivated vaccine with nanoencapsulation according to the invention can be of a size between about 20-500 nm, preferably between 50 and 100 nm, and most preferably about 80 nm. The concentration of virus of the inactivated vaccine without nanoencapsulation is more than 10⁶ TCID₅₀/ml, preferably between about 10⁶ and 10⁸ TCID₅₀/ml, and the most preferably about 10⁷ TCID₅₀/ml. The concentration of virus of the inactivated vaccine with nanoencapsulation is more than 10⁴ TCID₅₀/ml, preferably between about 10⁴ and 10⁶ TCID₅₀/ml, and the most preferably about 10⁵ TCID₅₀/ml. Furthermore, compared with conventional inactivated vaccine, the inactivated vaccine with nanoencapsulation according to the invention can achieve a similar protection effect with less amount, by saving at least 100-fold of dosage. Moreover, by applying nanoencapsultion, storage duration of the vaccine can be extended, for example, by 1 year at 4°C.

The inactivated vaccine and the nanoencapsulated inactivated vaccine according to the invention can be administrated via injection (for example, intraperitoneal injection, subcutaneously, and intramuscularly), oral administration, or bath-immunization, preferably via bath-immunization. The bath-immunization can be suitably applied on larvae whose body sizes are too small for injection, generally for larvae with age of less than one month. In the bath-immunization process, larvae are bath-immunized with the inactivated vaccine and the nanoencapsulated inactivated vaccine according to the invention at concentration of 10⁵-10⁷ TCID₅₀/ml at 20-25°C for 30-60 minutes.

The inactivated vaccine and the nanoencapsulated inactivated vaccine according to the invention can be used suitably for the immunization of general aquatic animals, such as fishes of sea water and fresh water, shrimps, or crabs, especially cultured fishes highly susceptible to NNV, for example, barramundi (*Lates Culcarifer*), sea bass (*Dicentrarchus labrax*), turbot (*Scophthalmus maximus*), red-spotted grouper (*Epinephelus akaara*), striped jack (*Pseudocaranx dentex*), Atlantic halibut (*Hippoglossus hippoglossus*), tiger puffer (*Takifugu rubripes*), Japanese flounder (*Opegnathus fasciatus* Temminck and Schlegel), barfin flounder (*Versaper moseri*), purplish amberjack (*Seriola dumerili*), sevenband grouper (*Epinephelus sepfemfasciatus*), Japanese sea bass (*Lateolabrax japonicus*) and the like.

These features and advantages of the present invention will be fully understood and appreciated from the following detailed description of the accompanying Drawings.

### BRIEF DESCRIPTION OF THE DRAWINGS

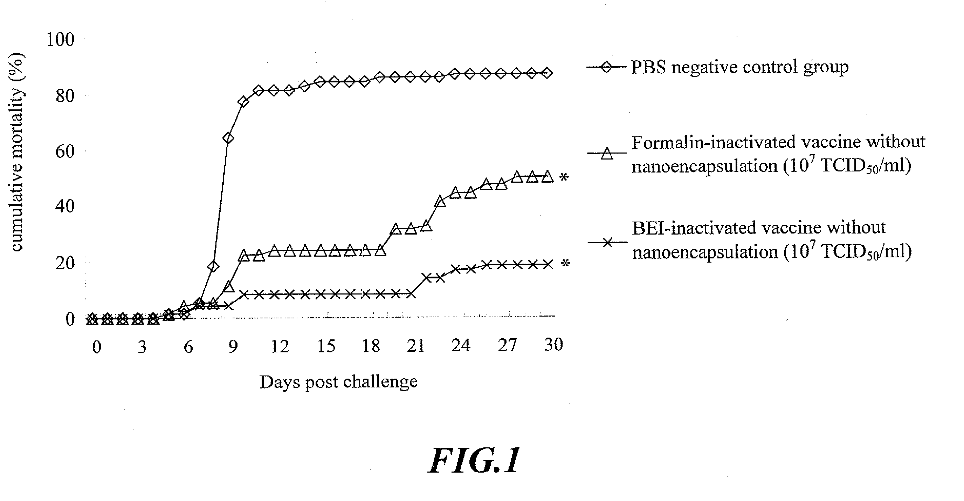

Figure 1 shows the cumulative mortalities of grouper larvae after bath challenge with NNV. Grouper larvae were bath-immunized with different reagents and then bath-challenged with NNV (1.6 × 10⁶ TCID₅₀/ml) 30 days post immunization, ◊ is referred to the cumulative mortality of grouper larvae bath-immunized with PBS as negative control group, Δ is referred to the cumulative mortality of grouper larvae bath-immunized with 0.2% formalin-inactivated NNV vaccine without nanoencapsulation (10⁷ TCID₅₀/ml), and × is referred to the cumulative mortality of grouper larvae bath-immunized with BEI-inaetivated NNV vaccine without nanoencapsulation (10⁷ TCID₅₀/ml). **p* < 0.001 (chi-square)

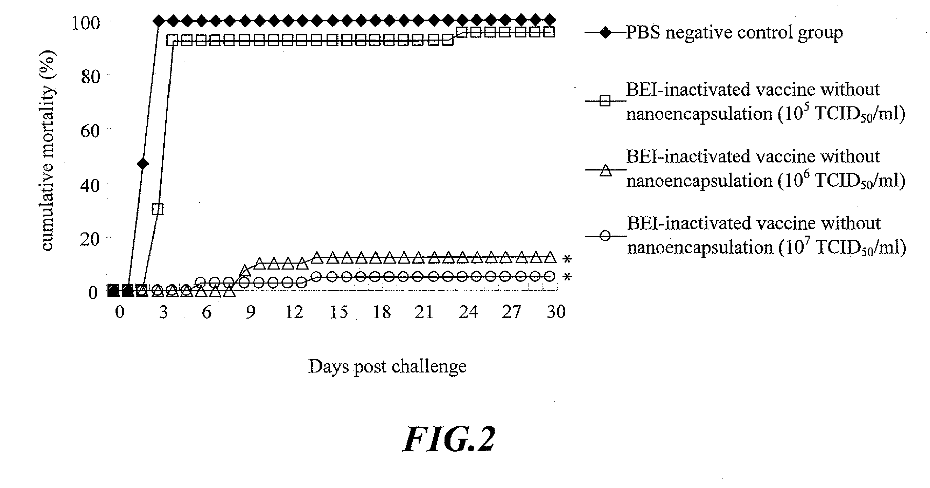

Figure 2 shows the cumulative mortalities of grouper larvae after bath challenge with NNV. Grouper larvae were bath-immunized with different reagents and then bath-challenged with NNV (1.6 × 10⁶ TCID₅₀/ml) 30 days post immunization. ◆ is referred to the cumulative mortality of grouper larvae bath-immunized with PBS as negative control group, □ is referred to the cumulative mortality of grouper larvae bath-immunized with BEI-inactivated NNV vaccine without nanoencapsulation (10⁵ TCID₅₀/ml), Δ is referred to the cumulative mortality of grouper larvae bath-immunized with BEI-inactivated NNV vaccine without nanoencapsulation (10⁶ TCID₅₀/ml), and ○ is referred to the cumulative mortality of grouper larvae bath-immunized with BEI-inactivated NNV vaccine without nanoencapsulation (10⁷ TCID₅₀/ml). **p* < 0.001 (chi-square)

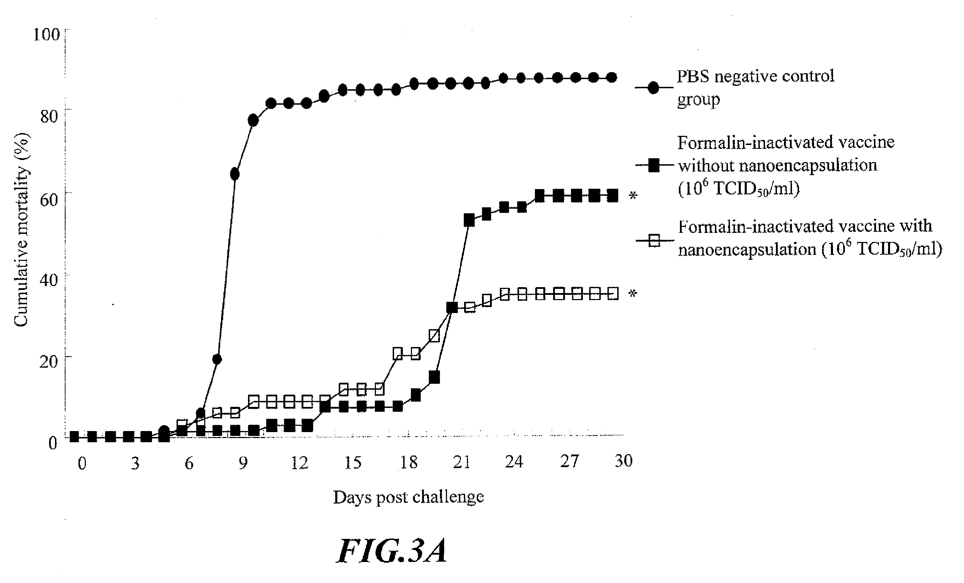

Figure 3A shows the cumulative mortalities of grouper larvae after bath challenge with NNV. Grouper larvae were bath-immunized with different reagents and then bath-challenged with NNV (1.6 × 10⁶ TCID₅₀/ml) 30 days post immunization. • is referred to the cumulative mortality of grouper larvae bath-immunized with PBS as negative control group, ■ is referred to the cumulative mortality of grouper larvae bath-immunized with 0.2% formalin-inactivated NNV vaccine without nanoencapsulation (10⁶ TCID₅₀/ml), and □ is referred to the cumulative mortality of grouper larvae bath-immunized with 0.2% formalin-inactivated NNV vaccine with nanoencapsulation (10⁶ TCID₅₀/ml). **p* < 0.001 (chi-square)

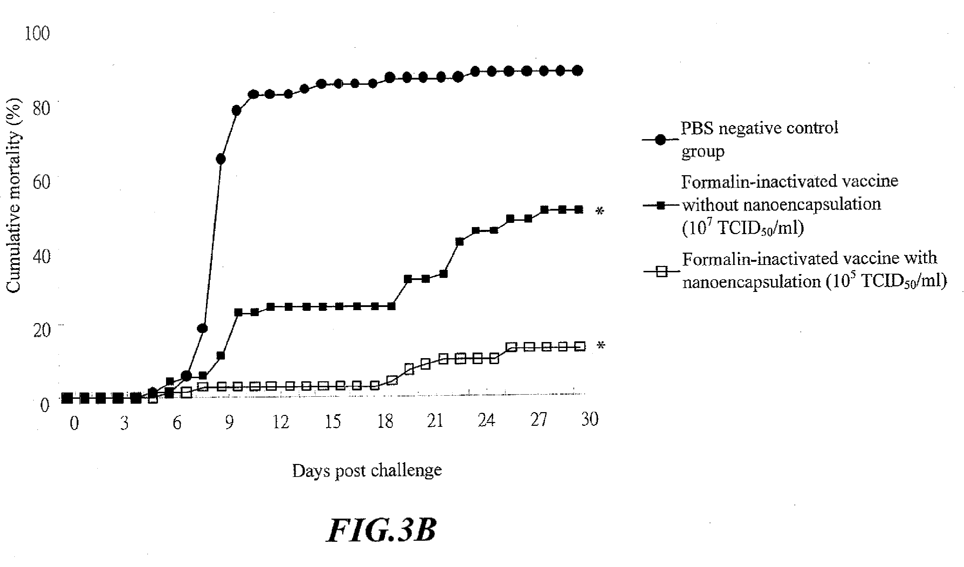

Figure 3B shows the cumulative mortalities of grouper larvae after bath challenge with NNV. Grouper larvae were bath-immunized with different reagents and then bath-challenged with NNV (1.6 × 10⁶ TCID₅₀/ml) 30 days post immunization. ● is referred to the cumulative mortality of grouper larvae bath-immunized with PBS as negative control group, ■ is referred to the cumulative mortality of grouper larvae bath-immunized with 0.2% formalin-inactivated NNV vaccine without nanoencapsulation (10⁷ TCID₅₀/ml), and □ is referred to the cumulative mortality of grouper larvae bath-immunized with 0.2% formalin-inactivated NNV vaccine with nanoencapsulation (10⁵ TCID₅₀/ml). **p* < 0.001 (chi-square)

### DETAILED DESCRIPTION OF THE PREFERRED EMBODIMENT

The invention will be illustrated more detail by following non-limiting examples.

### Example 1 Production of the aquatic nervous necrosis virus (NNV) vaccine

### 1. Amplification of virus

The GF-1 cells were infected with NNV, and then incubated at 28°C in L-15 broth (Leibovitz) supplemented with 2% fetal bovine serum (FBS) for 4 days. The culture fluid was then centrifuged at 2,000 g for 10 minutes, and the virus-containing supernatant was collected.

### 2. Inactivation of virus with formalin

Formalin stock solution (37%) was added into NNV-containing culture supernatant to reach a final concentration of 0.2%, and the mixture was incubated at 30°C for 3 days for inactivation. Thereafter, the mixture was dialyzed in phosphate buffer saline (PBS) at 4°C for 24 hours to get inactivated NNV solution. The inactivated NNV solution can be used directly as the aquatic nervous necrosis virus (NNV) vaccine. Alternatively, the inactivated NNV solution can be further nano-encapsulated by means of a nanoencapsulating process. The safety of each inactivated vaccine was examined by cell culture assay. The vaccines were confirmed to be completely inactivated by showing no viral titer in GF-1 cells after 3 serial passages, and were used for the following immunization tests.

### 3. Inactivation of virus with binary ethyleneimine (BEI)

Binary ethyleneimine (BEI) was used to inactivate nervous necrosis virus (NNV). 2.1 g of binary ethyleneimine (BEI) was added into 100 ml 0.175 N NaOH, and the mixture was heated in a water bath at 37°C for 2 hours to get binary ethyleneimine (BEI) solutuion. The BEI solution was added into the virus supernatant to reach a final concentration of 4 mM, and the mixture was incubated at 25°C for 3 days for inactivation. The mixture then was dialyzed in PBS at 4°C for 24 hours to get an inactivated NNV vaccine. The inactivated NNV supernatant can be used directly as the aquatic nervous necrosis virus (NNV) vaccine. Alternatively, the inactivated virus solution can be further nano-encapsulated by means of a nanoencapsulating process to get the aquatic nervous necrosis virus (NNV) vaccine.

### 4. Nanoencapsulation process

### (1) Formulation:

Base material: 60% by weight hydrogenated palm stearin and 30% by weight coconut oil.
Embedded substance: 10% by weight embedded substance. The embedded substance is the formalin-inactivated or BEI-inactivated virus solution mentioned above, and the inactivated virus solution was diluted 100-fold in PBS before the nanoencapsulation process.

### (2) Conditions:

Diameter of transporting tube: 20 mm
Flow rate: 150 l/h
Diameter of nozzle: 0.8 mm

The hydrogenated palm stearin, coconut oil and the inventive inactivated vaccine were homogeneously mixed under stirring at 55°C, and then the mixture was poured through transporting by a pump into a aqueous encapsulating solution at 38°C to form desired nanoencapsulated inactivated vaccine particles suspension which was then freeze-dried in a lyophilizer.

### (3) Results:

The average diameter of particles thus prepared was about 80 mm. The specific gravity of the aggregate of particles was less than that of water, and could be suspended in water. Its melting point was 43°C.

### Example 2 Fish anti-virus test 1- Efficacy trail: chemicals for inactivation of viral antigen

### 1. Fish for testing

A total number of 3000 orange-spotted grouper larvae (*Epinephelus coioides*) 40 days post hatchery (dph) with body weight (BW) of 0.2 g and total body length (TBL) of 2.4 cm were obtained from a private grouper hatchery farm of southern Taiwan. Before immunization, the fish were allowed to adapt for 1 week in a 400 liter-tank supplied with ozone-treated and re-circulated seawater, and fed with commercial dried pellets (Uni-President Enterprises Corp., Taiwan) six times a day. Ten fish were collected at random for NNV screening using RT-PCR and nested PCR. All samples were found to be negative.

### 2. Vaccination

Larvae were divided into two vaccine groups and one negative control group. Each group contained 100 larvae (n=100). Each group was kept in a separate 60 liter-aquarium prior to bath immunization and reared at 24-27°C. Larvae were bath-immunized with vaccines under following conditions:
Vaccine group 1: 0.2% formalin-inactivated vaccine without nanoencapsulation (the final concentration of NNV was 10⁷ TCID₅₀/ml).
Vaccine group 2: 4mM BEI-inactivated vaccine without nanoencapsulation (the final concentration of NNV was 10⁷ TCID₅₀/ml).
Negative control group: mock-immunized by PBS.
Bath-immunizations were carried out at temperature of 25°C for 30-60 minutes. After bath-immunization, larvae were transferred into a new tank.

### 3. Challenge test

Bath challenge tests were done 30 days post immunization. After immunized for 30 days, larvae were bath challenged at temperature of 25°C with NNV with a dosage of 1.6×10⁶ TCID₅₀/ml. Protection efficacy of vaccine was calculated on the base of relative percent survival (RPS) of immunized larvae as: RPS = 1 - (cumulative mortality of larvae in immunized group/cumulative mortality of larvae in control group) × 100%

### 4. Results:

To compare the efficacy of formalin-inactivated and BEI-inactivated vaccines, the final concentrations of the vaccines during bath immunization were adjusted to 10⁷ TCID₅₀/ml. There were no abnormalities in any of the vaccinated or control fish before challenge. The fish in each group were then bath-challenged 30 days post immunization. The cumulative mortality of the challenged fish was 87% for the negative control group, 50% for 0.2% formalin-inactivated vaccine group (RPS = 42.5%) and 19% for BEI-inactivated vaccine group (RPS = 78.2%) (Fig. 1). Moreover, statistically significant differences can be found between each vaccine group and the negative control group (*p*<0.001). The RPS value of the fish immunized by BEI-inactivated vaccine was 78.2%, which is much higher than that of the fish immunized by formalin-inaetivated vaccine (RPS = 42.5%). In addition, NNV was found in all the dead fish of each immunized group and negative control group after challenge test by RT-PCR examination.

### Example 3 Fish anti-virus test 2- Effective trail: dosage of vaccination

### 1. Fish for testing

The method was the same as example 2.

### 2. Vaccination

Larvae were divided into three vaccine groups and one negative control group. Each group contained 100 larvae (n=100). Each group was kept in a separate 60 liter-aquarium prior to bath immunization and reared at 24-27°C. Larvae were bath-immunized with vaccines under following conditions:
Vaccine group 1: 4mM BEI-inactivated vaccine without nanoencapsulation (the final concentration of NNV was 10⁵ TCID₅₀/ml).
Vaccine group 2: 4mM BEI-inactivated vaccine without nanoencapsulation (the final concentration ofNNV was 10⁶ TCID₅₀/ml).
Vaccine group 3: 4mM BEI-inactivated vaccine without nanoencapsulation (the final concentration of NNV was 10⁷ TCID₅₀/ml).
Negative control group: mock-immunized by PBS.
Bath-immunizations were carried out at temperature of 25°C for a time period of 30-60 minutes. After bath-immunization, larvae were caught off and placed in a new tank.

### 3. Challenge test

The method was the same as example 2.

### 4. Results:

The cumulative mortality of the fish in each group after NNV challenge is shown in Fig. 2. The RPS values of the fish bath-immunized with NNV vaccine with final concentration of 10⁵, 10⁶ and 10⁷ TCID₅₀/ml were 7.5%, 87.5% and 95%, respectively. Therefore, the minimal effective dosage of BEI-inactivated NNV vaccine for bath immunization is 10⁶ TCID₅₀/ml.

### Example 4 Fish anti-virus test 3- Efficacy trail: nanoencapsulation of the inactivated vaccine

### 1. Fish for testing

The method was the same as example 2.

### 2. Vaccination

Larvae were divided into four vaccine groups and one negative control group. Each group contained 100 larvae (n=100). Each group was kept in a separate 60 liter-aquarium prior to bath immunization and reared at 24-27°C. Larvae were bath-immunized with vaccines under following conditions:
Vaccine group 1: 2% Formalin-inactivated vaccine without nanoencapsulation (the final concentration of NNV was 10⁶ TCID₅₀/ml).
Vaccine group 2: 2% Formalin-inactivated vaccine with nanoencapsulation (the final concentration of NNV was 10⁶ TCID₅₀/ml).
Vaccine group 3: 2% Formalin-inactivated vaccine without nanoencapsulation (the final concentration of NNV was 10⁷ TCID₅₀/ml).
Vaccine group 4: 2% Formalin-inactivated vaccine with nanoencapsulation (the final concentration of NNV was 10⁵ TCID₅₀/ml).
Negative control group: mock-immunized by PBS.
Bath-immunizations were carried out at temperature of 25°C for a time period of 30-60 minutes. After bath-immunization, larvae were transferred into a new tank.

### 3. Challenge test

The method was the same as example 2.

### 4. Results:

The cumulative mortality of the fish in vaccine group 1 and 2 after NNV challenge is shown in Fig. 3A. At identical virus concentration of 10⁶ TCID₅₀/ml, the RPS values of the fish bath-immunized with nanoencapsulated vaccine (vaccine group 2) was 61%, while that of the fish bath-immunized with non-nanoencapsulated vaccine (vaccine group 1) was 32%.

The cumulative mortality of the fish in vaccine group 3 and 4 after NNV challenge is shown in Fig. 3B. The RPS value of the fish bath-immunized with nanoencapsulated vaccine at low virus concentration (10⁵ TCID₅₀/ml, vaccine group 4) was 85%, while that of the fish bath-immunized with non-nanoencapsulated vaccine at high virus concentration (10⁷ TCID₅₀/ml) was 43%. Therefore, the efficacy of NNV inactivated vaccine with nanoencapsultation is better than the efficacy of NNV inactivated vaccine without nanoencapsultation.

### Example 5 Fish anti-virus test 4- Efficacy trail: retention of protection induced by BEI-inactivated vaccine

### 1. Fish for testing

The method was the same as example 2.

### 2. Vaccination

Larvae were divided into three vaccine groups and three negative control group. Each group contained 100 larvae (n=100). Each group was kept in a separate 60 liter-aquarium prior to bath immunization and reared at 24-27°C. Larvae were bath-immunized with 4 mM BEI-inactivated vaccine without nanoencapulation (the final concentration of NNV was 10⁷ TCID₅₀/ml). Larvae in the negative control groups were mock-immunized by PBS. Bath-immunizations were carried out at temperature of 25°C for a time period of 30-60 minutes. After bath-immunization, larvae were transferred into a new tank. Bath challenge tests were done respectively on day 15, 30, and 90 post immunization with NNV at a challenge dosage of 1.6×10⁶ TCID₅₀/ml.

### 3. Challenge test

The method was the same as example 2.

### 4. Results:

Table 1 indicates that the cumulative mortality and RPS value of each group. The RPS value of the 15-day group was 30%, much lower than that of the 30-day group (RPS = 87.5%), indicating that 15 days were not enough for the larvae to develop efficient immunity against NNV infection. The RPS value of the 90-day group was 82%, as high as that of the 30-day group, indicating that efficient protection induced by immunization could last for at least 3 months.

**Table 1**

| | Cumulative mortality (RPS%) | | |
|---|---|---|---|
| Days post immunization | 15 days | 30 days | 90 days |
| BEI-inactivated vaccine | 43% (30%) | 12.5% (87.5%) | 10% (82%) |
| PBS | 61% | 100% | 55% |

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A method for preparing an aquatic nervous necrosis virus (NNV) vaccine, comprising following steps:
(a) culturing nervous necrosis viruses (NNV) by using a cell line to obtain a culture supernatant containing said nervous necrosis viruses (NNV), and
(b) treating said supernatant with an inactivating agent to form an aquatic nervous necrosis virus (NNV) vaccine.

2. A method for preparing an aquatic nervous necrosis virus (NNV) vaccine as recited in claim 1, wherein said cell line is any fish cell line susceptible to nervous necrosis virus (NNV).

3. A method for preparing an aquatic nervous necrosis virus (NNV) vaccine as recited in claim 1, wherein said cell line is one selected from the group consisting of GF-1, SSN-1, E-11, GB3, GF, GL, SISE, GS, SAF1 and GL-av.

4. A method for preparing an aquatic nervous necrosis virus (NNV) vaccine as recited in claim 1, wherein said virus is one selected from the group consisting of any NNV isolates separated from fish body with nervous necrosis disease.

5. A method for preparing an aquatic nervous necrosis virus (NNV) vaccine as recited in claim 1, wherein said inactivating agent is one selected from group consisting of formalin and binary ethyleneimine (BEI).

6. An aquatic nervous necrosis virus (NNV) vaccine, said vaccine is obtainable by the method as recited in claim 1.

7. A method for preparing an aquatic nervous necrosis virus (NNV) vaccine, comprising following steps:
(a) culturing nervous necrosis viruses (NNV) by using a cell line to obtain a supernatant containing said nervous necrosis viruses (NNV),
(b) treating said supernatant with an inactivating agent, and
(c) forming an aquatic nervous necrosis virus (NNV) vaccine from said inactivated supernatant by means of a nanoencapsulating process.

8. A method for preparing an aquatic nervous necrosis virus (NNV) vaccine as recited in claim 7, wherein the size of said vaccine is between about 20 and 500 nm.

9. A method for preparing an aquatic nervous necrosis virus (NNV) vaccine as recited in claim 7, wherein said cell line is any fish cell line susceptible to nervous necrosis virus (NNV).

10. A method for preparing an aquatic nervous necrosis virus (NNV) vaccine as recited in claim 7, wherein said cell line is one selected from the group consisting of GF-1, SSN-1, E-11, GB3, GF, GL, SISE, GS, SAF1 and GL-av.

11. A method for preparing an aquatic nervous necrosis virus (NNV) vaccine as recited in claim 7, wherein said virus is one selected from the group consisting of any NNV isolates separated from fish body with nervous necrosis disease.

12. A method for preparing an aquatic nervous necrosis virus (NNV) vaccine as recited in claim 7, wherein said inactivating agent is one selected from group consisting of formalin and binary ethyleneisnine.

13. An aquatic nervous necrosis virus (NNV) vaccine, said vaccine is obtainable by the method as recited in claim 7.
